(19) Europäisches Patentamt
European Patent Office
Office européen des brevets

(11) Numéro de publication : **0 192 931 B1**

(12) **FASCICULE DE BREVET EUROPÉEN**

(45) Date de publication du fascicule du brevet : **07.09.88**

(51) Int. Cl.⁴ : **C 25 B 3/02**, C 07 D261/00

(21) Numéro de dépôt : **86100220.2**

(22) Date de dépôt : **09.01.86**

(54) **Procédé pour la préparation d'isoxazoles.**

(30) Priorité : **25.01.85 CH 340/85**

(43) Date de publication de la demande : **03.09.86 Bulletin 86/36**

(45) Mention de la délivrance du brevet : **07.09.88 Bulletin 88/36**

(84) Etats contractants désignés : **CH DE FR GB LI NL**

(56) Documents cités :
**CH-A- 553 207**
**US-A- 4 297 181**
**ELECTROCHIMICA ACTA, vol. 30, no. 1, janvier 1985, pages 121-124, Pergamon Press Ltd., Oxford, GB; Z. OGUMI et al.: "Application of the SPE method to organic electro-chemistry-VI. Oxidation of cyclohexanol to cyclohexanone on Pt-SPE in the presence of iodine and iodide"**
**TETRAHEDRON LETTERS, vol. 22, no. 34, 1981, pages 3247-3248, Pergamon Press Ltd., Oxford, GB; H. NODA et al.: "Biomimetic oxidation of methyl 3,5-dibromo-4-hydroxyphenylpyruvate oxime and related phenols"**

(73) Titulaire : **FIRMENICH SA**
**1, route des Jeunes**
**CH-1211 Genève 8 (CH)**

(72) Inventeur : **Delay, François, Dr.**
**9, rue des Pervenches**
**CH-1227 Carouge (CH)**
Inventeur : **Joyeux, Michel, Dr.**
**23, chemin des Recluses**
**CH-1213 Petit-Lancy (CH)**

(74) Mandataire : **Salvadori, Giuseppe, Dr.**
**c/o Firmenich S.A. Case Postale 239**
**CH-1211 Genève 8 (CH)**

**EP 0 192 931 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention a trait à un procédé nouveau pour la préparation d'isoxazoles ou 1,2-oxazoles, composés caractérisés par la présence de l'unité structurelle que voici :

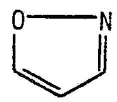

Plus particulièrement, les composés obtenus grâce à l'emploi du procédé de l'invention obéissent à la formule générale suivante :

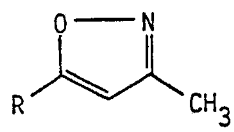

(I)

dans laquelle le symbole R sert à définir un reste aliphatique linéaire ou ramifié, saturé ou insaturé, cycloaliphatique ou aromatique, substitué ou non.

C'est ainsi que le symbole R peut représenter un alkyle linéaire $C_1$-$C_6$, ou ramifié tel un éthyle, propyle, n-butyle, iso-butyle, pentyle ou hexyle ; un alkyle insaturé tel le prop-1-ène-1-yle, le 2-méthyl-prop-1-ène-1-yle, le 1-éthyl-pent-1-ène-1-yle ou le 1-propyl-hex-1-ène-1-yle, par exemple ; un cycloalkyle, par exemple le 2,6,6-triméthyl-cyclohex-1-ène-1-yle ou le 2,6,6-triméthyl-cyclohex-2-ène-1-yle ; ou encore un phényle ou un 4-méthoxy-phényle.

Le procédé de l'invention est caractérisé par une réaction d'oxydation anodique d'une oxime alpha, bêta-insaturée en présence du couple $I_2/2I^{\ominus}$. Les oximes alpha,bêta-insaturées, utilisées comme produits de départ, obéissent à la formule suivante :

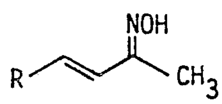

(II)

dans laquelle R est défini comme indiqué ci-dessus.

La synthèse des dérivés de type isoxazole a été déjà étudiée par le passé et nombreuses sont les méthodes de préparation documentées dans l'art antérieur, notamment dans Ber., 28, 2540 (1895) ; idem, 36, 3665 (1903) ; Compt. rend., 137, 795 (1903) ; J. Am. Chem. Soc., 49, 2078 (1927) ; Gazz. Chim. Ital. 70, 676 (1940) ; idem, 72, 99 (1942) ; idem, 76, 148 (1946). Les méthodes citées ci-dessus peuvent se différencier les unes des autres sur la base de la nature des produits de départ utilisés, à savoir :

1. réaction entre l'hydroxylamine et un dérivé 1,3-dicarbonylé :

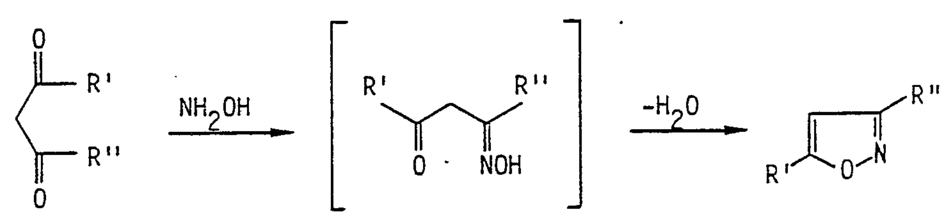


2. réaction entre l'hydroxylamine et une cétone ou un aldéhyde alpha-acétylénique :

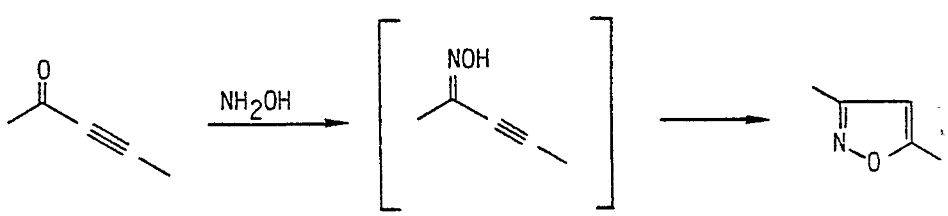


3. réaction entre l'hydroxylamine et une cétone ou un aldéhyde alpha,bêta-éthylénique halogéné :

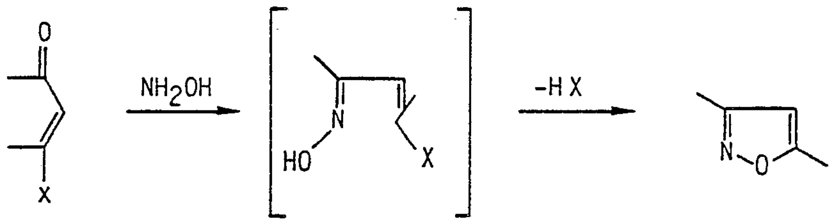


X = halogène

4. réaction entre l'hydroxylamine et une cétone ou un aldéhyde alpha,bêta-insaturé :

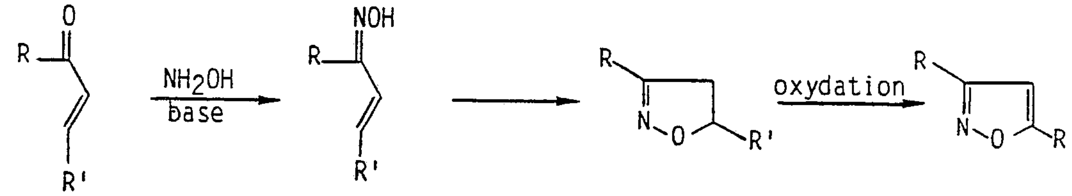


Le brevet suisse n° 553 207 propose un procédé pour la préparation d'isoxazoles de formule

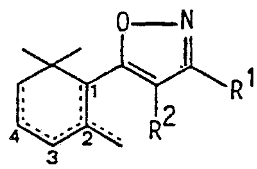

possédant une double liaison endocyclique en position 1, 2, 3 ou 4, ou une double liaison exocyclique en position 2, ou encore deux doubles liaisons conjuguées en positions 1 et 3, comme indiqué par les pointillés et dans laquelle $R^1$ et $R^2$ représentent un radical alkyle inférieure ou l'hydrogène, lequel procédé est caractérisé par la cyclisation d'une oxime alpha,bêta-insaturée de formule

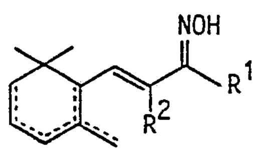

au moyen d'une quantité stoechiométrique de iode, de préférence en milieu neutre ou alcalin et en solution aqueuse. Pour maintenir en solution dans le milieu réactionnel l'iode requis, on suggérait l'adjonction d'iodure de potassium, lequel devait être employé à cet effet en large excès (3 ou 4 équivalents). Or, de tels réactifs sont relativement chers et leur emploi dans les quantités envisagées rend peu concurrentielle l'exploitation industrielle du procédé décrit.

La présente invention apporte une solution nouvelle à ce problème et propose un procédé électrochimique qui sert à pallier les inconvénients du procédé connu. Le procédé de l'invention est caractérisé en ce qu'on soumet à une oxydation anodique

a. un mélange hétérogène biphasique constitué par

(i) une solution d'une oxime alpha,bêta-insaturée de formule

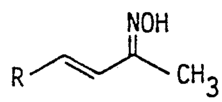 (II)

dans laquelle le symbole R sert à définir un reste aliphatique linéaire ou ramifié, saturé ou insaturé, cycloaliphatique ou aromatique, substitué ou non, dans le tétrahydrofuranne, et

(ii) une solution d'iodure de potassium et bicarbonate de sodium dans l'eau ; ou

b. un mélange homogène constitué par une solution de ladite oxime de formule (II), d'iodure de sodium et de bicarbonate de sodium dans le méthanol.

Le procédé de l'invention, caractérisé par un mode opératoire simple, offre plusieurs avantages par

rapport aux procédés connus. Il est apparu en effet que le déroulement de la réaction est facilement contrôlable, les rendements observés sont bons et l'opération est parfaitement reproductible, même dans des conditions variables de température, densité de courant ou concentration des réactifs. Il s'ensuit que son application à des préparations à l'échelle industrielle s'en trouve grandement facilité.

La concentration du produit de départ dans la solution ou mélange électrolytique n'est pas particulièrement critique. Toutefois, il est apparu que l'emploi de concentrations trop faibles réduit la rentabilité de l'opération, tandis que des concentrations trop élevées tendent à réduire la vitesse du processus d'oxydation désiré au profit de réactions secondaires, notamment lorsque le procédé a lieu dans un milieu hétérogène. Ce phénomène n'est que peu perceptible en milieu homogène. Dans l'un ou l'autre des systèmes, toutefois, il est préférable de ne pas dépasser une concentration en oxime de départ d'environ 10 %.

Quant aux électrodes qui peuvent être employées dans le procédé de l'invention, elles peuvent consister en plaquettes de graphite ou en feuilles de platine pour ce qui est de l'anode et feuilles de platine ou en plaquettes de titane, de Monel (marque enregistrée) (alliage de nickel et cuivre) ou de nickel pour ce qui est de la cathode. Une anode de type DSA (marque enregistrée de Diamond Shamrock Technologies SA) en oxyde métallique, sous forme de grille, pourrait également être envisagée.

Il est apparu que le système graphite (anode) / titane (cathode) était préférentiel.

Les densités de courant utilisées étaient de l'ordre de 10 à 50 mA/cm$^2$. Toutefois, l'influence de ce paramètre sur le déroulement de la réaction n'est pas déterminante.

La température de la réaction peut être comprise entre environ 25 et 70 °C, de préférence la réaction est effectuée à la température d'ébullition du système choisi ou à une température voisine de celle-ci.

Le procédé selon l'invention peut être conduit en présence d'air, mais de préférence sous atmosphère d'un gaz inerte, l'azote ou l'argon par exemple.

Comme il a été précisé plus haut, la réaction est effectuée en présence de bicarbonate de sodium. Une très petite quantité de ce dernier était nécessaire et suffisante à la bonne marche de la réaction. Alors que l'utilisation de plus grandes quantités restait sans effet, l'absence totale de bicarbonate de sodium entraîne un ralentissement prononcé de la réaction, accompagné de la formation de produits secondaires à haut poids moléculaire. En milieu homogène, 1 à 2 % de bicarbonate de sodium suffisent.

La concentration du iodure alcalin, iodure de potassium dans le cas de la réaction en milieu hétérogène ou iodure de sodium dans le cas de la réaction en milieu homogène, peut varier dans une gamme assez large. Dans les deux cas toutefois, 30 % en poids semble être la concentration minimum requise.

Quant à la proportion de tétrahydrofuranne (THF) dans le mélange THF/$H_2O$, dans le cas de la réaction en milieu hétérogène, elle n'a pas d'effet critique. Par conséquent, l'on préférera utiliser le système le plus économique, donc une concentration réduite en THF. La présence de ce dernier est cependant nécessaire, faute de quoi la réaction sera accompagnée de la formation d'un important résidu.

Le procédé peut être conduit dans un électrolyseur à cuve unique ou à cuve séparée et il peut fort bien se prêter à une mise en œuvre continue et, à cet effet, une cellule de type tubulaire semble parfaitement convenir.

L'invention est illustrée par l'un des exemples suivants dans lesquels les températures sont indiquées en degrés centigrades et les abréviations ont le sens usuel dans l'art.

## Exemple 1

3-Méthyl-5-[2,6,6-triméthyl-cyclohex-1-ène-1-yl]-isoxazole (méthode a.)

3,5 g (17 mM) de l'oxime de la bêta-ionone dont la pureté est de 97 % ont été dissous dans 35 ml de tétrahydrofuranne et la solution résultante a été ajoutée à la solution aqueuse constituée par 20 g d'iodure de potassium (120 mM) et 1 g (12 mM) de bicarbonate de soude dans 15 ml d'eau.

Le mélange, sous atmosphère d'argon, est agité et porté à reflux (env. 65°) et une densité de courant de 50 mA/cm$^2$ est alors appliquée. La réaction a lieu dans un récipient standard tricol de 100 ml doté d'un rôdage mâle normalisé en téflon faisant office de support aux deux élecrodes distantes de 1 cm. Le réacteur comprend également un refrigérant, un thermomètre ainsi qu'une entrée et une sortie pour la circulation d'argon. La solution est agitée magnétiquement.

Le mélange devient brun foncé et le déroulement de la réaction est suivi par analyse gazchromatographique. Après 4 h d'électrolyse, ce qui résulte à env. 90 % de conversion, le courant est coupé et le reflux est maintenu jusqu'à décoloration du mélange (2 à 3 h). La totalité de l'oxime a alors réagi. Après refroidissement à température ambiante, la phase organique est séparée, séchée et évaporée.

Le résidu est distillé à pression réduite (13,3 Pa) pour fournir 2,9 g (14,5 mM) de l'isoxazole désiré à Eb. 80-85°. Au cours de l'électrolyse, le pH de la solution passe progressivement de 8,1 à 10,2 où il se stabilise. La solution aqueuse peut être réutilisée telle quelle pour l'oxydation d'une nouvelle charge d'oxime dans le THF.

## Exemple 2

3-Méthyl-5-[2,6,6-triméthyl-cyclohex-1-ène-1-yl]-isoxazole (méthode b.)

La réaction a été effectuée dans un réacteur d'électrolyse analogue à celui qui a été décrit à l'exemple précédent.

3,5 G d'oxime de bêta-ionone (17 mM), 20 g d'iodure de sodium (133 mM) et 1 g (12 mM) de bicarbonate de sodium ont été versés dans 50 ml de méthanol et le mélange résultant a été porté à reflux (env. 60°) sous agitation et sous atmosphère d'argon. L'électrolyse procède comme décrit à l'exemple 1 en appliquant une densité de courant de 50 mA/cm$^2$. Après refroidissement à température ambiante, le solvant a été évaporé et le résidu visqueux qui en résulte a été repris dans 250 ml de toluène. De telle sorte l'iodure de sodium qui était en solution précipite et il est récupéré quantitativement par filtration. Le filtrat a été ensuite concentré et le résidu a été distillé sous vide (13,3 Pa). On a ainsi obtenu 2,96 g (14,5 mM) de l'isoxazole désiré ayant Eb. 80-85°/13,3 Pa. Rendement : 85 %. L'iodure de sodium récupéré peut être utilisé pour des opérations d'électrolyse successives.

L'isoxazole obtenu était en tous points identique à celui décrit dans le brevet US n° 3,931,323.

En opérant de façon identique à celle qui a été décrite ci-dessus, on a préparé les isoxazoles suivants de formule (I)

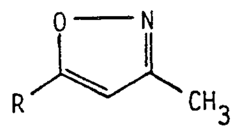

(I)

à partir des oximes alpha,bêta-insaturées de formule (II)

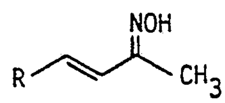

(II)

Tableau

| | R | Eb. [°C/Pa] |
|---|---|---|
| 1. | $CH_3-(CH_2)_3$ | 120/1,33x10$^3$ |
| 2. | $(CH_3)_2-CH-(CH_2)_2$ | 130/1,33x10$^3$ |
| 3. | $(CH_3)_2-C=CH$ | 145/1,33x10$^3$ |
| 4. | $CH_3-(CH_2)_2-CH=C$<br>$C_2H_5$ | 110-130/1,33 |
| 5. | $CH_3-(CH_2)_3-CH=C$<br>$C_3H_7$ | 92-99/1,33 |
| 6. | $C_6H_5$ | 125/1,33 |
| 7. | p-$CH_3O-C_6H_4$ | 101-103 (F) |
| 8. | furyl | 170/1,33x10$^3$ |

Les isoxazoles ainsi obtenus présentaient les caractères analytiques suivants :

1. 3-méthyl-5-butyl-isoxazole

IR (film) : 2 960, 1 610, 1 420 $cm^{-1}$ ;
RMN (360 MHz ; $CDCl_3$) : 0,93 (3H, t) ; 1,64 (2H, q) ; 1,88 (2H, sextuplet) ; 2,24 (3H, s) ; 2,70 (2H, t), 5,79 (1H, s) deltq ppm ;
SM : $M^+$ = 139 (20) ; m/e : 110 (8), 97 (100), 82 (25), 68 (9), 55 (57), 41 (24).

2. 3-méthyl-5-(3-méthylbutyl)-isoxazole

IR (film) : 2 990, 1 620, 1 460 $cm^{-1}$ ;
RMN (360 MHz ; $CDCl_3$) : 0,92 (6H, d) ; 1,55 (3H, m) ; 2,25 (3H, s) ; 2,70 (2H, t) ; 5,79 (1H, s) delta ppm ;
SM : $M^+$ = 153 (2) ; m/e : 112 (28), 97 (100), 82 (33), 69 (29), 55 (78), 43 (58), 41 (56).

3. 3-méthyl-5-(2-méthylprop-1-ène-1-yl)-isoxazole

IR ($CHCl_3$) : 3 000, 1 665, 1 593, 1 587, 1 420 $cm^{-1}$ ;
RMN (360 MHz ; $CDCl_3$) : 1,95 (3H, s) ; 2,01 (3H, s) ; 2,27 (3H, s) ; 5,90 (1H, s) ; 6,13 (1H, s) delta ppm ;
SM : $M^+$ = 137 (100), m/e : 122 (7), 108 (10), 95 (39), 83 (63), 67 (48), 55 (85), 53 (41), 41 (37), 39 (68).

4. 3-méthyl-5-(hept-3-ène-3-yl)-isoxazole

IR (film) : 2 980, 1 650, 1 585, 1 425 $cm^{-1}$ ;
RMN (360 MHz ; $CDCl_3$) : (isomère trans)- : 0,94 (3H, t) ; 1,07 (3H, t) ; 1,48 (2H, sextuplet) ; 2,19 (2H, q) ; 2,27 (3H, s) ; 2,39 (2H, t) ; 5,94 (1H, s) ; 6,27 (1H, t) delta ppm ;
RMN (360 MHz ; $CDCl_3$) : (isomère cis)- : 0,94 (3H, t) ; 1,07 (3H, t) ; 1,48 (2H, s) ; 2,19 (2H, q) ; 2,31 (3H, s) ; 2,39 (2H, t) ; 5,19 (1H, t), 5,98 (1H, s) delta ppm ;
SM : (isomère trans)- : $M^+$ = 179 (44) ; m/e : 164 (6), 150 (38), 137 (100), 122 (33), 109 (35), 96 (51), 81 (79), 67 (36), 55 (49), 41 (57) ;
SM : (isomère cis-) : $M^+$ = 179 (28) ; m/e : 164 (3), 150 (45), 137 (100), 122 (33), 109 (51), 96 (52), 81 (77), 67 (48), 55 (49), 41 (62).

5. 3-méthyl-5-(non-4-ène-4-yl)-isoxazole

IR (film) : 2 950, 1 640, 1 580, 1 450, 780 $cm^{-1}$ ;
RMN (360 MHz ; $CDCl_3$) : (isomère trans-) : 0,91 (3H, t) ; 0,93 (3H, t) ; 1,35-1,55 (3 × 2H, m) ; 2,21 (2H, q) ; 2,26 (3H, s) ; 2,35 (2H, t) ; 5,92 (1H, s) ; 6,41 (1H, t) delta ppm ;
RMN (360 MHz ; $CDCl_3$) : (isomère cis-) : 0,91 (3H, t) ; 0,93 (3H, t) ; 1,35-1,55 (3 × 2H, m) ; 2,21 (2H, q) ; 2,29 (3H, s) ; 2,35 (2H, t) ; 5,69 (1H, t) et 5,98 (1H, s) delta ppm ;
SM : (cis = trans) : $M^+$ = 207 (16) ; m/e : 178 (3), 164 (18), 151 (10), 136 (12), 123 (100), 110 (9), 95 (22), 82 (17), 67 (19), 55 (14), 41 (23).

6. 3-méthyl-5-phénylisoxazole

IR ($CHCl_3$) : 3 040, 1 610, 1 600, 1 580, 1 420, 1 210 $cm^{-1}$ ;
RMN (360 MHz ; $CDCl_3$) : 2,36 (3H, s) ; 6,35 (1H, s) ; 7,43 (3H, m) ; 7,75 (2H, m) delta ppm ;
SM : $M^+$ = 159 (100) ; m/e : 130 (14), 116 (6), 105 (51), 89 (12), 82 (18), 77 (48), 63 (7), 51 (25).

7. 3-méthyl-5-(4-méthoxyphényl)-isoxazole

IR ($CHCl_3$) : 3 010, 1 620, 1 515, 1 255, 1 175, 910 $cm^{-1}$ ;
RMN (360 MHz ; $CDCl_3$) : 2,33 (3H, s) ; 3,85 (3H, s) ; 6,24 (1H, s) ; 6,96 (2H, d), 7,69 (2H, d) delta ppm ;
SM : $M^+$ = 189 (100) ; m/e : 174 (15), 160 (1), 146 (18), 135 (49), 119 (11), 107 (8), 92 (15), 77 (24), 63 (13), 51 (10), 41 (4).

8. 3-méthyl-5-(2-furyl)-isoxazole

IR ($CHCl_3$) : 3 000, 1 643, 1 625, 1 545, 1 450, 1 415, 1 010, 890, 790 $cm^{-1}$ ;
RMN (360 MHz ; $CDCl_3$) : 2,32 (3H, s) ; 6,26 (1H, s) ; 6,52 (1H, m) ; 6,84 (1H, d), 7,51 (1H, s) delta ppm ;
SM : $M^+$ = 149 (100) ; m/e : 120 (2), 106 (4), 95 (43), 82 (32), 66 (18), 52 (15), 39 (18).

L'oxime de la bêta-ionone, utilisée comme produit de départ dans les exemples 1 et 2 ci-dessus, peut être préparée conformément au procédé décrit dans le brevet US 3,931,323 [voir en particulier l'exemple 3]. De manière analogue, on a procédé à la préparation des oximes de formule (II) à partir des cétones correspondantes.

**Revendications**

1. Procédé pour la préparation d'un isoxazole de formule

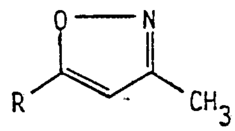

(I)

dans laquelle le symbole R sert à définir un reste aliphatique linéaire ou ramifié, saturé ou insaturé, cycloaliphatique ou aromatique, substitué ou non, caractérisé en ce qu'on soumet à une oxydation anodique

a. un mélange hétérogène biphasique constitué par

(i) une solution d'une oxime alpha,bêta-insaturée de formule

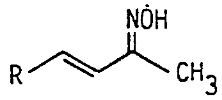

(II)

dans laquelle le symbole R a le sens indiqué ci-dessus, dans le tétrahydrofuranne, et

(ii) une solution d'iodure de potassium et bicarbonate de sodium dans l'eau ; ou

b. un mélange homogène constitué par une solution de ladite oxime de formule (II), d'iodure de sodium et de bicarbonate de sodium dans le méthanol.

2. Procédé suivant la revendication 1, caractérisé en ce que la réaction s'effectue en utilisant une anode en platine ou en graphite.

3. Procédé suivant la revendication 1, caractérisé en ce que la réaction s'effectue en utilisant une cathode en titane, en nickel ou en alliage nickel-cuivre.

4. Procédé suivant les revendications 2 et 3, caractérisé en ce que la réaction s'effectue à l'aide d'une anode en graphite et d'une cathode en titane.

5. Procédé suivant l'une des revendications 1 à 4, caractérisé en ce que la concentration de l'oxime de formule (II) dans le milieu réactionnel est égale ou inférieure à 10 %.

6. Procédé suivant l'une des revendications 1 à 5, caractérisé en ce qu'on utilise comme oxime de formule (II) la bêta-ionone oxime et que l'on obtient le 3-méthyl-5-(2,6,6-triméthyl-cyclohex-1-ène-1-yl)-isoxazole.

**Claims**

1. Process for the preparation of an isoxazole of formula

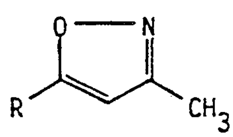

(I)

wherein symbol R represents a linear or branched, saturated or unsaturated, aliphatic radical, or a cycloaliphatic or a substituted or unsubstituted aromatic radical, charaterized in that

a. a two-phase heterogeneous mixture consisting of

(i) a solution of an alpha,beta-unsaturated oxime of formula

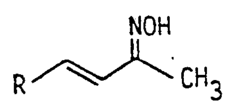

(II)

wherein symbol R has the meaning given above, in tetrahydrofurane, and

(ii) a solution of potassium iodide and sodium bicarbonate in water ; or

b. a homogeneous mixture consisting of a solution of the said oxime of formula (II), of sodium iodide and of sodium bicarbonate in methanol

is subjected to an anodic oxidation.

2. Process according to claim 1, characterized in that the reaction is effected by using a platinum or a graphite anode.

3. Process according to claim 1, characterized in that the reaction is effected by using a titanium, a nickel or a nickel-copper alloy cathode.

4. Process according to claims 2 and 3, characterized in that the reaction is effected by means of a graphite anode and a titanium cathode.

5. Process according to any of claims 1 to 4, characterized in that the concentration of the oxime of formula (II) in the reaction medium is equal to or lower than 10 %.

6. Process according to any of claims 1 to 5, characterized in that there is used as oxime of formula (II) beta-ionone oxime and there is obtained 3-methyl-5-(2,6,6-trimethyl-cyclohex-1-en-1-yl)-isoxazole.

**Patentansprüche**

1. Verfahren zur Herstellung eines Isoxazoles der Formel

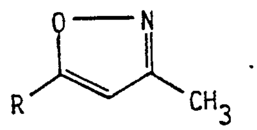

(I)

in welcher das Symbol R einen linearen oder verzweigten, gesättigten oder ungesättigten aliphatischen Rest, einen cycloaliphatischen oder substituierten oder unsubstituierten aromatischen Rest darstellt, dadurch gekennzeichnet, dass man

a. ein heterogenes Zweiphasengemisch bestehend aus

(i) einer Lösung eines alpha,beta-ungesättigten Oxims der Formel

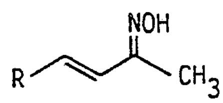

(II)

in welcher das Symbol R die oben angegebene Bedeutung hat, in Tetrahydrofuran, und

(ii) einer Lösung von Kaliumiodid und Natriumbicarbonat in Wasser, oder

b. eine homogene Mischung bestehend aus einer Lösung des oben genannten Oxims der Formel (II), Natriumiodid und Natriumbicarbonat in Methanol

einer anodischen Oxydation unterwirft.

2. Verfahren nach Anspruch 1, dadurch gekennzeichnet, dass die Reaktion unter Verwendung einer Platin- oder Graphitanode stattfindet.

3. Verfahren nach Anspruch 1, dadurch gekennzeichnet, dass die Reaktion unter Verwendung einer Kathode aus Titan, Nickel, oder einer Nickel-Kupferlegierung stattfindet.

4. Verfahren nach Ansprüchen 2 und 3, dadurch gekennzeichnet, dass die Reaktion unter Verwendung einer Graphitanode und einer Titankathode stattfindet.

5. Verfahren nach einem der Ansprüche 1-4, dadurch gekennzeichnet, dass die Konzentration des Oxims der Formel (II) im Reaktionsmittel 10 % oder weniger beträgt.

6. Verfahren nach einem der Ansprüche 1-5, dadurch gekennzeichnet, dass man als Oxim der Formel (II) beta-Iononoxim verwendet, und 3-Methyl-5-(2,6,6-trimethyl-cyclohex-1-en-1-yl)-isoxazol erhält.